# EUROPEAN PATENT APPLICATION

(11) **EP 1 698 321 A1**
(43) Date of publication of application: **06.09.2006**
(21) Application number: 04808030.3
(22) Date of filing: 21.12.2004
(51) Int. Cl.: A61K 8/18

(54) **HAIR COSMETIZER AND PROCESS FOR PRODUCING THE SAME**

(30) Priority: 24.12.2003 JP 2003427267
(71) Applicant: Sakakibara, Itsuo, Nagoya-shi Aichi 4660812 (JP)
(72) Inventor: Sakakibara, Itsuo, Nagoya-shi Aichi 4660812 (JP)
(74) Representative: TBK-Patent
(86) International application number: PCT/JP2004/019679
(87) International publication number: WO 2005/060925

(57) **Abstract**

A hair cosmetizer of the present invention is such that the hair cosmetizer including an oily material, which is solid at ordinary temperature, is dissolved into water or is dispersed therein by compounding a water-soluble organic medium with a cosmetic material being the major component of the hair cosmetizer. And, since the water-soluble organic medium is fastened firmly to hair by means of the adhesion force of the oily material, which is solid at ordinary temperature, the water-soluble organic medium as a moisturizing material becomes less likely to be eliminated, and thereby the effects, such as gloss and moisture, sustain for a prolonged period of time. A production process of the present invention for a hair cosmetizer can produce the hair cosmetizer of the present invention, which is good in terms of water solving and which can be applied readily, by mixing a material, which includes a heat-melted oily material and a powdery water-soluble organic medium, to prepare a mixture, and forming the post-cooling-and-solidifying mixture as a powdery shape by pulverizing it.

## Description

### TECHNICAL FIELD

The present invention relates to a hair cosmetizer, which gives gloss, moisture, and the like, to hair.

### BACKGROUND ART

As for hair cosmetizers for giving gloss and moisture to hair, those, such as hair conditioners and hair packs, for example, have been known (Patent Literature 1, for instance). These hair cosmetizers are those which are completed by compounding various materials in order to give gloss and moisture to hair.

For example, in the case of compounding a moisturizing material with a hair cosmetizer, the moisturizing material adheres onto the surface of hair in such a state that it contains water, or goes into the damaged parts of hair so that water is provided for hair and thereby moisture is given thereto. However, since the moisturizing material is eliminated from hair with ease by shampooing, and the like, there has been a problem in that the effect does not sustain for a prolonged period of time.

Meanwhile, in the case of compounding an oily material with a hair cosmetizer, gloss is given to hair. However, since the oily material is such that the viscosity is high, it is extremely difficult to coat the small amount on hair uniformly. When coating the oily material excessively, there has been a problem in that there occur such drawbacks that hair has gotten sticky, hairs have been entangled with each other, and so forth.

It is possible as well to think of coating a small amount of an oily material on hair uniformly by dissolving the oily material into a solvent or dispersing it therein to coat it together with the solvent. As described above, since a moisturizing material is employed in such a state that it contains water, it is preferable that an oily material is dissolved into water or is dispersed therein. However, since an oily material is not provided with any hydrophilic group, there has been a problem in that it dose not dissolve into water or disperse therein as in its own state.
[Patent Literature 1]
Japanese Unexamined Patent Publication (KOKAI) No. 2003-026,542

### DISCLOSURE OF THE INVENTION

(Assignment to be solved by the Invention)

The present invention has been done while taking the aforementioned circumstances into consideration, and it is an object to provide a hair cosmetizer, in which the effects, such as gloss and moisture, sustain for a prolonged period of time and at the same time which can be coated on hair substantially uniformly, and a process for producing the same.

(Means for Solving the Assignment)

A hair cosmetizer of a first invention, which solves the aforementioned assignment, is characterized in that a cosmetic material including an oily material, which is solid at ordinary temperature, and a water-soluble organic medium is the major component; and it dissolves into water or disperses therein.

This cosmetic material can preferably be powdery.

A hair cosmetizer of a second invention, which solves the aforementioned assignment, is characterized in that a cosmetic material including an oily material, which is solid at ordinary temperature, and a water-soluble organic medium is the major component; and the cosmetic material is dissolved into water or is dispersed therein.

In the hair cosmetizers of the first invention and second invention, the aforementioned oily material can preferably be rosin, and the aforementioned water-soluble organic medium can preferably be carboxymethylcellulose.

Further, in the hair cosmetizers of the first invention and second invention, the aforementioned water-soluble organic medium can preferably be included in a range of 10-100 parts by weight with respect to 100 parts by weight of the aforementioned oily material.

A hair-cosmetizer production process of a third invention is for producing the hair cosmetizer of the first invention, and is characterized in that a material, which includes a heat-melted oily material and a powdery water-soluble organic medium, is mixed to prepare an oily-material mixture, and the mixture is formed as a powdery shape by pulverizing it after cooling it to solidify.

In the hair-cosmetizer production process of the third invention, the aforementioned powdery water-soluble organic medium can preferably be such that the average particle diameter is 180 µm or less.

(Effect of the Invention)

The hair cosmetizers of the fist invention and second invention are those in which the cosmetic material is the major component. The cosmetic material is one which includes the water-soluble organic medium as a moisturizing material, and the oily material, which is solid at ordinary temperature. In the hair cosmetizers of the present inventions, the water-soluble organic medium, which is included in the cosmetic material, works as a moisturizing material, but this water-soluble organic medium is fastened to hair by means of the adhesive force of the oily material, which is included in the cosmetic material similarly. Since the oily material in the present inventions is one which is solid at ordinary temperature, and demonstrates a high adhesive force, the water-soluble organic medium is fastened firmly to hair so that it becomes less likely to be eliminated, and thereby the effects, such as gloss and moisture, sustain for a prolonged period of time.

Moreover, to the hair cosmetizers of the present inventions, since the water-soluble organic medium is added together with the oily material, the contacting area of the oily material with respect to hair becomes small. Therefore, the oily material does not adhere onto hair excessively, and thereby such drawbacks that hair has gotten sticky by means of the adhesive force of the oily material, hairs have been entangled with each other, and so forth, disappear.

Further, the hair cosmetizer of the first invention of the present inventions dissolves into water or disperses therein. Therefore, the hair cosmetizer of the first invention of the present inventions can be used by dissolving it into water (or by dispersing it therein), and thereby it becomes possible to coat the hair cosmetizer onto hair substantially uniformly.

Note that this cosmetic material can take various shapes, such as aggregate shapes and powdery shapes, however, in the case of turning it into a powdery shape especially, there is an advantage in that it becomes likely to be dissolved into water or be dispersed therein.

The hair cosmetizer of the second invention of the present inventions is one in which the cosmetic material is dissolved into water or is dispersed therein to complete it, and includes the hair cosmetizer of the first invention and water. Since the hair cosmetizer of the second invention is such that the cosmetic material is dissolved into water or is dispersed therein in advance, the time and labor for dispersing it in water or dissolving it thereinto when being in use have been saved so that it becomes a readily usable form. And, since the cosmetic material and water become familiar with each other in advance, the concentration unevenness of the cosmetic material, and the like, disappear, and thereby it can be coated onto hair more uniformly.

### BEST MODE FOR CARRYING OUT THE INVENTION

The hair cosmetizers according to the first invention and second invention of the present inventions are such that a cosmetic material including an oily material, which is solid at ordinary temperature, and a water-soluble organic medium, is adapted to the major component. As for the cosmetic material, it is possible to use those, which can give gloss, and the like, to hair and at the same time which is solid at ordinary temperature and demonstrates a high adhesive force at ordinary temperature, for example, it is possible to use rosin (pine resin), or those, such as Vaseline, animal fat, wax and paraffin, preferably. Among them, since rosin is good in terms of the adhesive force so that it can fasten the water-soluble organic medium onto hair firmly, it is used especially preferably.

As for the water-soluble organic medium, it is possible to use those in which they themselves hold water so that they work as a moisturizing material, for example, it is possible to use carboxymethylcellulose, and the like, preferably. Since carboxymethylcelluose is good in terms of the water absorption action and can hold a large amount of water, it is used especially preferably.

The reason why the hair cosmetizer of the first invention dissolves into water or disperses therein is not clear, however, it is assumed as follows.

The cosmetic material, being the major component of the present inventions, includes, the oily material, which is solid at ordinary temperature, and the water-soluble organic medium. Among these, since the water-soluble organic medium is a material, which dissolves into water, a property of dispersing in water or dissolving thereinto partially, property which derives from the water-soluble organic material, is given to the cosmetic material, in which the oily material and water-soluble organic medium are included. Moreover, since the oily material bonds with the water-soluble organic medium by means of its own adhesion force, it disperses into water together with the water-soluble organic medium. By means of these, it is believed that, despite the fact that the hair cosmetizers of the present inventions include the oily material, which is insoluble to water, they dissolve into water or disperse therein.

Here, the amount of the water-soluble organic medium, which is included in the hair cosmetizers of the first invention and the second invention, can preferably be in a range of 10-100 parts by weight with respect to 100 parts by weight of the oily material. When the amount of the water-soluble organic medium exceeds 100 parts by weight, since the amount of the oily material with respect to the water-soluble organic medium does not become sufficient, it becomes difficult to securely fasten the water-soluble organic material to hair by means of the oily material. Moreover, when the amount of the water-soluble organic medium is less than 10 parts by weight, there is the case of not giving moisture to hair sufficiently.

Moreover, in order to let the hair cosmetizers go into the damaged portions of hair, the less the average diameter of the cosmetic material, which includes the water-soluble organic medium and the oily material, is, the more preferable it is, and it can preferably be 180 µm or less.

With the hair cosmetizers of the present inventions, it is possible to compound at least one member of ammonium thioglycollate, L-cysteine hydrochloride, monoethanolamine, cetanol and lauryl sulfate. By further adding at least one member of these components, the hair cosmetizers are fastened more firmly to hair so that they become less likely to be eliminated, and thereby the effects, such as gloss and moisture, come to sustain a more prolonged period of time.

In the hair cosmetizers of the present inventions, it is possible to compound known materials having been used in hair cosmetizers, known materials which are represented by coloring materials, fragrant materials, antiseptic agents, antioixdants, ultraviolet-ray absorbing agents, and the like. In this case, effects other than the effects, which result from the oily material and water-soluble organic solvent, are given.

Moreover, it is advisable to further compound a treatment agent, such as diethanolamide stearate, cetyltrimethlyammonium chloride, cetanol,, polyoxyethylene cetylether, propylen glycol, lactic acid and horse oil. In this case, the treatment effects other than the effects, such as gloss and moisture, which result from the oily material and water-soluble organic medium, can be given to the hair cosmetizers.

The hair-cosmetizer production process of the present invention is a process for producing the hair cosmetizer of the first invention. This production process of the hair cosmetizer is one in which a material, which includes a heat-melted oily material and a powdery water-soluble organic medium, is mixed to prepare a mixture, and the obtained mixture is formed as a powdery shape by pulverizing it after cooling it to solidify.

In the case of adding a water-soluble organic medium to a solid-state oily substance to mix, it is very difficult to mix the oily material with the water-soluble organic medium substantially uniformly, and accordingly there is a case where the unevenness of components occurs in the obtained hair cosmetizer. In this instance, desired moisture and gloss cannot be obtained, which has resulted from the unevenness of components. There is more than that, that is, there is a case where the water-soluble organic medium becomes likely to be eliminated from hair so that the moisture, which results from the water-soluble organic medium, does not sustain for a prolonged period of time. Further, because of the fact that the oily material is present excessively, there occurs such a drawback that hair has gotten sticky, hairs have been entangled with each other, or the like.

By adding a powdery water-soluble organic medium to a heat-melted oily material to mix, a hair cosmetizer whose components are free from unevenness can be obtained, and thereby the above-described various drawbacks come not to occur. And, by forming the obtained mixture as a powdery shape by pulverizing it after cooling it to solidify, since the superficial area per volume of the hair cosmetizer enlarges, a hair cosmetizer, which is good in terms of the water solubleness and is readily usable, can be obtained.

Note that, since the oily material becomes solid at ordinary temperature, the mixture solidifies at ordinary temperature. Therefore, in the case of adapting the mixture to a hair cosmetizer as it is, for example, it takes a very long time to dissolve it into water. Moreover, in addition to heating the mixture once to melt the oily material during application, for instance, it is possible to dissolve it into water, in this case, however, very complicated operations become necessary when applying the hair cosmetizer.

Here, as described above, the smaller the average particle diameter of a powder, which includes the water-soluble organic medium and the oily material, the more preferable it is, however, it becomes difficult to make the average particle diameter of a powder, which includes the water-soluble organic medium and the oily material, smaller, depending on the shape of the water-soluble organic medium. Therefore, it is needed to use a powdery one as for the water-soluble organic medium, and it is preferable to use one whose average particle diameter is 180 µm or less.

(Example)

Hereinafter, the hair cosmetizer of the present invention and the production process for the same will be described while naming an example.

The hair cosmetizer of the present example is one which includes rosin as the oily material, which is solid at ordinary temperature, and carboxymethylcellulose as the water-soluble organic medium. The hair cosmetizer of the present example is produced as follows.

100-parts-by-weight rosin was weighed out, and was melted by heating. To the melted rosin, 30-parts-by-weight carboxymethylcellulose of 180-µm average particle diameter was added, and was mixed for 20 minutes under the conditions of 20 revolutions per 1 minute at 180 °C using a heating iron pot. The obtained mixture was cooled to room temperature, and thereby a solid-state mixture was obtained.

The obtained solid-state mixture was pulverized with a pulverizer, and thereby a powdery hair cosmetizer of 360-µm average particle diameter was obtained.

(Functional Test)

The hair cosmetizer obtained in the example was coated onto the hair of 58 subjects, and the sustainable period of gloss and moisture was examined. The coating of the hair cosmetizer was carried out in the following manner.

First of all, 120-parts-by-weight water was added to 100-parts-by-weight hair cosmetizer obtained in the example, and was mixed well. The obtained mixture of the hair cosmetizer and water was coated onto the subjects' hair, and was warmed. At this moment, since a large amount of the rosin was adhered onto the hair, the squeaking, which derived from the rosin, occurred in the hair. After the warming, the hair with the hair cosmetizer coated was washed with a shampooing agent. The excessive rosin, which adhered to the hair surface, was removed by this washing, and thereby the squeaking was got rid of. When it was dried after the washing, good gloss and moisture were given to the hair.

At a later date, the number of days during which the subjects felt that the gloss and moisture of the hair sustained was surveyed. As a result, it was found out that 15 of the subjects felt that they sustained for 15 days-20 days; 35 of the subjects felt that they sustained for 20 days-30 days; and 8 of the subjects felt that they sustained for 25 days-35 days. These are a very long number of days with respect to the fact that the effect of gloss and moisture by means of ordinary cosmetizers sustains for approximately one day (shampooing once) only. From this functional test as well, it is understood that the gloss and moisture by means of the hair cosmetizer of the present invention sustain for a very prolonged period of time.

## Claims

1. A hair cosmetizer being **characterized in that** a cosmetic material including an oily material, which is solid at ordinary temperature, and a water-soluble organic medium is the major component; and it dissolves into water or disperses therein.

2. The hair cosmetizer set forth in claim 1, wherein said cosmetic material is powdery.

3. The hair cosmetizer set forth in claim 1, wherein said water-soluble organic medium is carboxymethylcellulose.

4. The hair cosmetizer set forth in claim 1, wherein said water-soluble organic medium is included in a range of 10-100 parts by weight with respect to 100 parts by weight of said oily material.

5. A hair cosmetizer being **characterized in that** a cosmetic material including an oily material, which is solid at ordinary temperature, and a water-soluble organic medium is the major component; and the cosmetic material is dissolved into water or is dispersed therein.

6. The hair cosmetizer set forth in claim 5, wherein said oily material is rosin.

7. The hair cosmetizer set forth in claim 5, wherein said water-soluble organic medium is carboxymethylcellulose.

8. The hair cosmetizer set forth in claim 5, wherein said water-soluble organic medium is included in a range of 10-100 parts by weight with respect to 100 parts by weight of said oily material.

9. A process being for producing a hair cosmetizer, in which a cosmetic material including an oily material, which is solid at ordinary temperature, and a water-soluble organic medium is the major component; and it dissolves into water or disperses therein, the hair cosmetizer production process being **characterized in that**:
a material, which includes a heat-melted oily material and a powdery water-soluble organic medium, is mixed to prepare a mixture, and the mixture is formed as a powdery shape by pulverizing it after cooling it to solidify.

10. The process for producing a hair cosmetizer set forth in claim 9, wherein said powdery water-soluble organic medium is such that the average particle diameter is 180 µm or less.
